# FASCICULE DE BREVET EUROPEEN

(11) **EP 0 956 008 B1**
(45) Date de publication et mention de la délivrance du brevet: **16.03.2005**
(21) Numéro de dépôt: 97944941.0
(22) Date de dépôt: 08.10.1997
(51) Int. Cl.: A61K 9/70, A61K 7/00

(54) **MATERIAU SUPPORT DE MICROCAPSULES**
TRÄGERMATERIAL FÜR MIKROKAPSELN
MICRO-CAPSULE SUPPORT MATERIAL

(30) Priorité: 11.10.1996 FR 9612920
(43) Date de publication de la demande: 17.11.1999
(73) Titulaire: 3X Engineering, 98000 Monaco (MC)
(72) Inventeur: BOULET D'AURIA, Stanislas, F-06320 Cap d'Ail (FR)
(74) Mandataire: Bonneau, Gérard
(86) Numéro de dépôt international: PCT/FR1997/001793
(87) Numéro de publication internationale: WO 1998/016207

(56) Documents cités:
- DE-A- 3 447 833
- GB-A- 1 304 375
- US-A- 3 918 452
- US-A- 4 828 542
- DATABASE WPI Week 9339 Derwent Publications Ltd., London, GB; AN 93-309281 XP002033736 & JP 05 222 231 A (INOAC CORP KK) , 31 août 1993

## Description

### Domaine technique

La présente invention concerne un matériau support de microcapsules destinées à contenir des produits devant être conservés à l'abri de l'air avant utilisation, ainsi que les applications d'un tel matériau dans le domaine pharmaceutique, cosmétique et des parfums.

### Etat de la technique

Dans le domaine cosmétique et également pharmaceutique, beaucoup de produits liquides sont vendus en flacons. Dès que le flacon est ouvert pour être utilisé, il se produit forcément une oxydation par contact avec l'air et donc une altération du produit contenu dans le flacon. Il est donc souvent impossible de continuer à utiliser le produit qui a perdu une partie de ses propriétés dans un temps assez court.

D'autres produits sont vendus sur un support comme c'est le cas des lingettes pré-imprégnées d'eau de toilette ou de parfum. Outre le fait qu'une fois l'emballage ouvert, ces supports deviennent des nids de bactéries, ils se dessèchent ensuite très rapidement. Ces inconvénients existent également dans le cas des lingettes de démaquillage déjà pré-imprégnées d'un lait pour démaquillage.

On a donc songé à utiliser des microbilles ou microcapsules collées sur un support en papier ou autre. Il est donc nécessaire de frotter ou gratter le support pour faire éclater les microcapsules et libérer le produit contenu. En fait, ce type de support ne se prête pas à une utilisation commerciale et est réservé à la présentation d'échantillons dans une revue ou un support publicitaire.

Une autre technique, tel que décrite, dans le document US-A-4828452, consiste à utiliser un matériau support de microcapsules dans lequel les microcapsules sont disséminées dans un liant solide de consistance spongieuse de manière à ce que le ou les produits contenus dans les microcapsules soient libérés lorsqu'une pression est exercée sur le support. Le liant solide est un polymère obtenu à partir d'un mélange fluide de produits de polymérisation. Les microcapsules sont versées dans le mélange sous agitation, au cours de la polymérisation et avant solidification, permettant ainsi une répartition uniforme des microcapsules. Malheureusement, lorsque l'on se contente d'utiliser un matériau dans lequel les microcapsules sont disséminées de façon uniforme, il s'avère que seulement une faible partie du produit (par exemple 10%) contenu dans les microcapsules par exemple un produit pharmaceutique, est libérée lors de son utilisation. Comme la plupart du temps, ce type de produit est jetable, il y a donc un gaspillage très important du produit qui entraîne un coût prohibitif.

### Exposé de l'invention

C'est pourquoi le but de l'invention est de fournir un matériau support de produit liquide ou en poudre destiné à une application pharmaceutique, cosmétique ou autre, qui soit d'utilisation pratique et qui remédie aux inconvénients énoncés ci-dessus.

Un autre but de l'invention est de fournir un matériau support de microcapsules permettant une libération maximale du produit contenu dans les microcapsules lors de l'utilisation du matériau.

L'objet de l'invention est donc un matériau support de microcapsules contenant au moins un produit actif devant être conservé à l'abri de l'air, les microcapsules étant disséminées dans un liant solide de consistance spongieuse de manière à ce que le produit actif soit libéré lorsqu'une pression est exercée sur le matériau support ; les microcapsules étant réparties dans le matériau support de manière à ce qu'en s'éloignant de la surface d'application, soit la taille des microcapsules diminue, soit la quantité de microcapsules par unité de volume diminue linéairement, de manière à ce que la quantité de produit actif par unité de volume diminue au fur et à mesure qu'on s'éloigne de ladite surface d'application, permettant ainsi que la quantité de produit actif libéré au niveau de ladite surface d'application soit maximale lors de la première utilisation.

### Description brève des dessins

Les buts, objets et caractéristiques de l'invention apparaîtront clairement à la lecture de la description qui suit faite en référence aux dessins dans lesquels :
la figure 1 représente schématiquement un bloc de matériau support selon l'invention,
la figure 2 est un diagramme représentant la quantité de produit actif par unité de volume lorsqu'on s'éloigne de la surface d'application du bloc de la figure 1,
la figure 3 représente schématiquement un récipient contenant de la mousse après expansion due à la polymérisation,
la figure 4 représente le même récipient de mousse que celui de la figure 3, mais avec un couvercle sur lequel est exercée une force,
la figure 5 est une coupe de gélule utilisée comme applicateur du matériau selon l'invention,
la figure 6 est une illustration de la gélule de la figure 5 au moment de son utilisation, et
la figure 7 représente un exemple de dispositif d'application conforme aux principes de l'invention.

La technique des microcapsules est bien connue dans le domaine biomédical. Les microcapsules sont des particules sphériques, constituées d'une enveloppe solide contenant un liquide, un solide ou une substance pâteuse.

Chaque microcapsule qui peut avoir une dimension s'échelonnant entre 50µm et 1,5mm constitue donc un réservoir proprement dit. La microencapsulation dont la première application industrielle a en fait été la mise au point du papier carbone sans carbone (carbonless paper), a permis de résoudre un grand nombre de problèmes en pharmacie. En effet, les microcapsules, grâce à leur membrane qui limite les échanges avec l'extérieur, assurent une protection du médicament vis-à-vis des différents agents chimiques ou physiques tels que l'humidité, la chaleur ou l'oxydation. En outre, les microcapsules permettent de contrôler la vitesse de libération des principes actifs qui y sont contenus et donc de moduler leur biodisponibilité. Ceci peut être réalisé en agissant sur un nombre important de paramètres technologiques : nature du matériau constituant la membrane, proportion des principes actifs, taille des particules, épaisseur de l'enveloppe des microcapsules.

Toutes les propriétés mentionnées ci-dessus ont été mises à profit pour l'administration de médicaments à ingérer. L'invention tire profit en partie de ces propriétés en disséminant les microcapsules dans un support à consistance spongieuse dans le but d'une utilisation externe des produits contenus dans les microcapsules.

Un mode de réalisation de l'invention consiste à réaliser le matériau support de microcapsules à l'aide d'une mousse telle qu'une mousse de polyuréthane, une mousse de polyéthylène ou autre polymère ayant une consistance spongieuse. La mousse de polyuréthane, par exemple, est fabriquée de façon classique par polycondensation en mélangeant un polyol et de l'isocyanate en présence d'eau qui provoque un dégagement gazeux de dioxyde de carbone et donc la formation de cellules qui donne un aspect de mousse de type spongieux au polymère résultant. Les microcapsules sont ajoutées aux trois composants mentionnés ci-dessus et le tout est mélangé. Quand la polycondensation est terminée, le produit obtenu est donc un matériau spongieux comportant des microcapsules disséminées dans la masse de la mousse qui sert de liant.

Une des caractéristiques du matériau de l'invention est que les cellules communiquent les unes avec les autres pour que les produits contenus dans les microcapsules puissent s'écouler lors de l'utilisation du matériau. Il faut donc mettre une quantité d'eau suffisante pour provoquer la formation de cellules ouvertes (c'est à dire communiquant entre elles). L'idéal est bien sûr d'avoir un matériau comportant 100% de cellules ouvertes, mais les applications mentionnées ci-dessous sont possibles si on a affaire à une mousse comportant seulement 60% de cellules ouvertes. En effet, en plus de la diffusion par l'intérieur des cellules, il s'avère qu'il existe également une diffusion des produits par imprégnation et par capillarité.

Dans le mélange des ingrédients nécessaires pour fabriquer le matériau support de l'invention, le pourcentage en poids de microcapsules peut varier énormément entre moins de 1% lorsque les produits contenus dans les microcapsules sont très actifs à faible dose, à 60% dans certains cas. Ce pourcentage peut d'ailleurs varier selon la nature du matériau support.

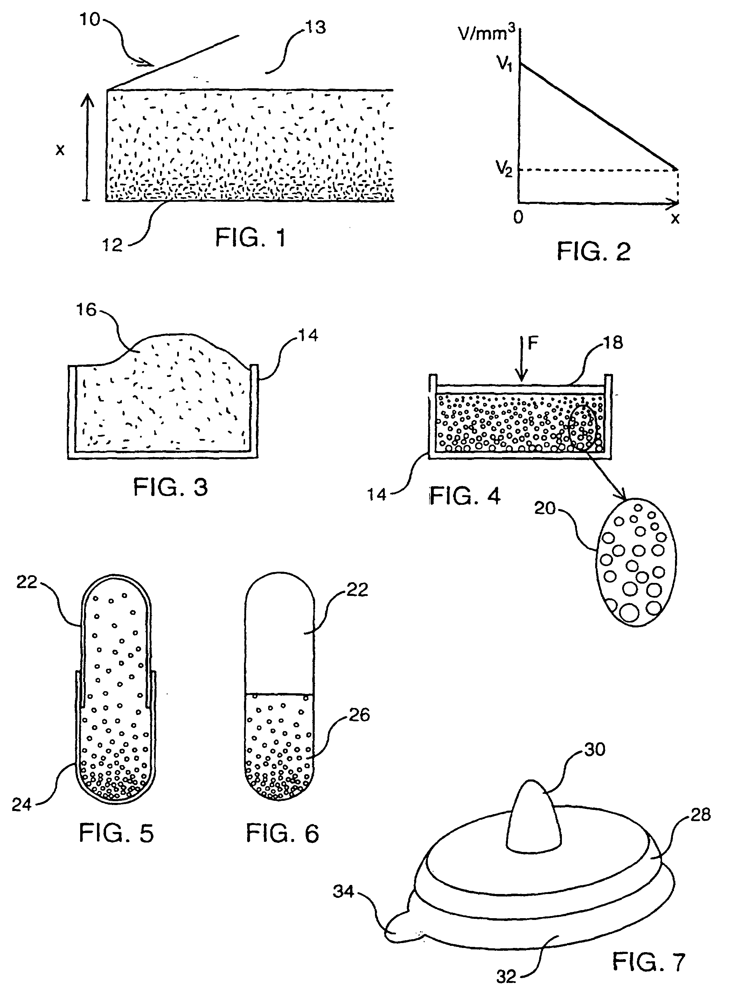

La caractéristique essentielle de l'invention est que le produit actif contenu dans les microcapsules soit dans une proportion qui diminue lorsqu'on s'éloigne de la surface d'application. Il y a deux façons d'obtenir cette caractéristique. Soit les microcapsules sont de taille identique et on fait en sorte que le nombre de microcapsules par unité de volume diminue lorsqu'on s'éloigne de la surface d'application. Ceci est illustré sur la figure 1 représentant un bloc 10 de matériau support tel que de la mousse renfermant des microcapsules dont la densité par unité de volume diminue lorsqu'on s'éloigne d'une distance x par rapport à la surface d'application 12. Comme on le voit sur le diagramme de la figure 2, le volume de produit actif par mm³ décroît d'une valeur V₁ à une valeur V₂ lorsque la distance passe de 0 à x. Bien que la décroissance de V soit linéaire dans le mode de réalisation préféré, toute autre répartition décroissante pourrait être utilisée.

Une autre façon d'obtenir le résultat ci-dessus est de diminuer la taille des microcapsules par exemple de 1,2mm à 0,05mm lorsqu'on s'éloigne de la surface d'application tout en gardant homogène la répartition volumique des microcapsules à l'intérieur du matériau support. Que ce soit l'une ou l'autre technique qui est utilisée, elle sont relativement aisées à mettre en oeuvre lors de la fabrication du matériau.

Grâce à cette répartition décroissante du produit actif lorsqu'on s'éloigne de la surface d'application, la quantité de produit actif libéré lorsqu'une pression sera exercée de haut en bas sur le bloc 10 de la figure 1, sera maximale. Ainsi, on pourra obtenir une libération de 70% du produit actif alors que 10% seulement sont libérés s'il y a répartition uniforme du produit actif. Ce résultat est essentiel dans tous les cas où le produit est utilisé une seule fois et est ensuite jeté.

Une alternative pour obtenir une libération maximale de produit actif est de faire varier la grosseur des cellules. Plus les cellules ont un volume important, plus le produit actif (liquide en général) s'écoule facilement et en outre l'effort d'écrasement à fournir pour obtenir cette libération est moindre. Ce résultat peut être obtenu très facilement lors de la fabrication. En effet, si pendant l'élaboration de la mousse on laisse ouvert le récipient 14 où sont versés les composants destinés à la polycondensation (par exemple polyol et isocyanate), comme illustré sur la figure 3, il y a expansion de la mousse 16 qui prend la forme illustrée tout en obtenant une répartition uniforme des microcapsules à l'intérieur de la mousse. Si par contre, on applique un couvercle plat 18 avec force F sur la mousse en expansion comme illustré sur la figure 4, la répartition des cellules ou bulles d'air n'est plus uniforme. Les cellules sont de plus en plus importantes en volume au fur et à mesure que l'on descend vers le fond du récipient 14 comme le montre l'agrandissement 20 d'une portion de la mousse.

Les applications du matériau support selon l'invention sont très nombreuses. Ainsi, une application importante consiste à préparer des gélules assez grosses (diamètre 5 mm) remplies d'une mousse préparée comme décrit ci-dessus et contenant des microcapsules remplies de produits pharmaceutiques à application cutanée, des produits désinfectants aseptisants, ou encore de produits cosmétiques. La gélule représentée sur la figure 5 est maintenue enfermée dans un étui formé d'une première partie 22 et d'une deuxième partie 24. La gélule est ensuite écrasée par pression entre les doigts de façon à faire éclater les microcapsules. Puis, la gélule est ouverte par retrait de la deuxième partie 24 (voir figure 6) de l'étui et l'application du ou des produits actifs sur la peau peut être réalisée facilement en tenant la partie 22 non ouverte de la gélule et en pressant le matériau spongieux sur les endroits voulus. Contrairement au flacon habituel pour lequel il faut d'abord imprégner un tissu ou un tampon en coton avec le risque de se répandre du produit sur les doigts, le produit (liquide dans ce cas) est maintenu dans la mousse et n'est exsudé que lorsqu'on exerce la pression pour l'appliquer sur la peau.

Dans l'application ci-dessus du type gélule jetable après première utilisation, il est nécessaire d'obtenir la libération de tout le produit actif immédiatement. Une manière de réaliser les gélules est d'utiliser des microcapsules de tailles différentes. Elle sont versées dans un des deux produits de polymérisation et un mixage vigoureux est effectué juste avant l'effet de « moussage » dû à la polymérisation. Ainsi, les petites microcapsules restent en haut alors que les grosses microcapsules descendent à la partie inférieure. La densité volumique du produit actif est donc plus importante dans la portion 26 utilisée de la gélule que dans la portion se trouvant enfermée dans la première partie 22 de l'étui tenu entre les doigts lors de l'application du produit.

Le matériau support de microcapsules selon l'invention peut aussi remplacer avantageusement les lingettes pré-imprégnées de parfum, d'eau de toilette ou de liquide de démaquillage. Tant qu'on n'a pas exercé une pression sur le support qui peut en la circonstance avoir une forme quelconque, les produits contenus dans les microcapsules ne risquent pas de s'altérer. Il n'est pas nécessaire de présenter ce support sous emballage, par exemple un emballage métallisé comme c'est le cas des lingettes d'eau de toilette distribuées dans les avions.

Un applicateur de produit actif entrant dans le cadre des applications selon l'invention est illustré sur la figure 7. La fabrication d'un tel applicateur se fait dans un moule. La partie en mousse 28 contenant les microcapsules comporte une portion de préhension 30 servant à saisir l'ensemble. Avant utilisation, le matériau en mousse 28 adhère à une partie sécable 32. L'utilisateur commence par malaxer le matériau 28 pour libérer le plus possible de produit actif, avant de retirer la partie sécable 32 grâce à la languette 34. Le retrait de la partie 32 permet de dégager la surface d'application. De la même façon que pour les gélules, la quantité de produit actif (nombre de microcapsules ou leur taille) devra être plus importante près de la surface d'application recouverte avant utilisation par la partie sécable 32.

Dans le domaine de l'hygiène, on peut envisager d'utiliser un matériau selon l'invention contenant du savon ou un produit de toilette à l'intérieur des microcapsules comme par exemple des gants de toilette jetables après utilisation.

Enfin, il est également possible d'utiliser un matériau support de microcapsules comme dispensateur d'effluves odorantes, notamment de parfum contenu dans les microcapsules. Il suffira d'exercer une pression à un endroit du dispensateur présenté sous la forme d'une éponge, pour laisser un peu de parfum s'exhaler. Cette manipulation pourra être répétée plusieurs fois jusqu'à ce que toutes les microcapsules disséminées dans l'éponge aient été écrasées.

Une autre application consiste utiliser le matériau support de microcapsules selon l'invention en tant que coussin, notamment pour chien ou chat, un tel coussin contenant des microcapsules remplies d'un produit à caractère insecticide (par exemple un produit anti-puces) ou attractif.

Toujours dans le cadre de l'invention, il est possible d'utiliser un matériau support de microcapsules comme applicateur de produit actif durable. Dans ce cas, contrairement à l'exemple de la gélule qui ne sert qu'une fois, il faut que la quantité de produit actif libéré soit à peu près constante à chaque application. Ceci peut être aisément obtenu en utilisant le bloc de matériau 10 de la figure 1 de manière inverse, c'est à dire que la surface d'application est la surface supérieure 13 du bloc et non plus la surface inférieure 12. Dans ce cas, au fur et à mesure des applications au moyen d'une pression exercée sur la surface 12, le produit actif libéré à la surface d'application 13 proviendra des couches de matériau de plus en plus profondes, c'est à dire des couches contenant de plus en plus de microcapsules. Par conséquent, la difficulté croissante pour le produit actif d'atteindre la surface d'application, sera compensée par la quantité croissante de produit actif provenant des couches supérieures du produit (ici en se rapprochant de la surface 12) ce qui permettra une libération de quantité constante de produit actif à condition d'appliquer une pression plus important au fur et à mesure des utilisations.

Bien d'autres applications de l'invention sont encore possibles dans d'autres domaines inattendus comme par exemple la pêche à la ligne où le support sous forme de boulettes imprégnées de microcapsules contenant l'appât est écrasé avec les doigts avant d'être lancé dans l'eau.

Il est donc clair que le matériau support de microcapsules selon l'invention peut être utilisé dans une multitude d'applications où le produit à appliquer ou à utiliser ne doit être libéré qu'au moment de son utilisation. En outre, on peut envisager des applications dans lesquelles il existe deux sortes de microcapsules contenant deux produits différents dont les contenus entrent en réaction chimique au moment où la pression est exercée sur le support comme par exemple une réaction de polymérisation dans le cas d'une colle à deux composants (une résine époxyde).

## Revendications

1. Matériau support (10) de microcapsules contenant au moins un produit actif devant être conservé à l'abri de l'air, lesdites microcapsules étant disséminées dans un liant solide de consistance spongieuse de manière à ce que le produit actif soit libéré lorsqu'une pression est exercée sur le matériau support ;
ledit matériau étant **caractérisé en ce qu'**en s'éloignant de la surface d'application (12), soit la taille des microcapsules diminue, soit la quantité de microcapsules par unité de volume diminue linéairement, de manière à ce que la quantité de produit actif par unité de volume diminue au fur et à mesure qu'on s'éloigne de ladite surface d'application, permettant ainsi que la quantité de produit actif libéré au niveau de ladite surface d'application soit maximale lors de la première utilisation.

2. Matériau (10) selon la revendication 1, dans lequel la quantité de produit actif libéré lors de la première utilisation est égale à 70% de la quantité totale du produit actif contenu dans le matériau.

3. Matériau (10) selon la revendication 1 ou 2, dans lequel la quantité de produit actif par unité de volume décroît linéairement lorsqu'on s'éloigne de la surface d'application (Fig. 2).

4. Matériau (10) selon la revendication 1, 2, ou 3, dans lequel la quantité de microcapsules de même taille diminue linéairement lorsqu'on s'éloigne de la surface d'application (12).

5. Matériau support de microcapsules selon l'une des revendications 1 à 4 dans lequel ledit liant de consistance spongieuse est une mousse de polymère contenant au moins 60% de cellules ouvertes.

6. Matériau support de microcapsules selon la revendication 5, dans lequel ladite mousse est une mousse de polyuréthane.

7. Utilisation du matériau support de microcapsules selon l'une des revendications 1 à 6, pour obtenir un médicament destiné à un traitement cutané utilisant le produit contenu dans lesdites microcapsules.

8. Utilisation du matériau support de microcapsules selon l'une des revendications 1 à 6, comme dispositif applicateur d'un produit cosmétique contenu dans lesdites microcapsules.

9. Utilisation du matériau support de microcapsules selon l'une des revendications 1 à 6, comme dispositif dispensateur d'effluves odorantes et notamment de parfum contenu dans lesdites microcapsules.

10. Utilisation du matériau support de microcapsules selon l'une des revendications 1 à 6, comme dispensateur de savon contenu dans lesdites microcapsules.

11. Gélule (22, 24, 26) contenant un matériau support de microcapsules pour mettre en oeuvre l'utilisation selon l'une des revendications 7 à 10.

12. Matériau support (10) de microcapsules contenant au moins un produit actif devant être conservé à l'abri de l'air, lesdites microcapsules étant disséminées dans un liant solide de consistance spongieuse de manière à ce que le produit actif soit libéré lorsqu'une pression est exercée sur le matériau support ;
ledit matériau étant **caractérisé en ce que** la quantité de microcapsules par unité de volume ou la taille des microcapsules augmentent en s'éloignant de la surface d'application (13) de manière à ce que la quantité de produit actif par unité de volume augmente au fur et à mesure qu'on s'éloigne de ladite surface d'application permettant ainsi que la quantité de produit actif libéré au niveau de ladite surface d'application soit à peu près constante à chaque utilisation.

13. Matériau support de microcapsules selon l'une des revendications 1 à 6 ou 12, dans lequel certaines des microcapsules contiennent un premier produit et les autres microcapsules contiennent un deuxième produit, les deux produits entrant en réaction lorsqu'une pression est exercée sur le support.

14. Utilisation du matériau support de microcapsules selon la revendication 13, comme colle résultant d'une réaction chimique entre les deux produits contenus dans lesdites microcapsules.

## Claims

1. A microcapsule support material (10) containing at least one active product not to be exposed to air, said microcapsules being disseminated in a solid binder having a spongy consistency such that the active product is released when pressure is exerted on the support material;
said material being **characterised in that**, when there is movement away from the application surface (12), either the size of the microcapsules decreases, or the quantity of microcapsules per unit of volume decreases in a linear fashion, such that the quantity of active product per unit of volume decreases as there is movement away from said application surface, thus enabling the quantity of active product released at said application surface to be at a maximum at the first usage.

2. The material (10) according to Claim 1, in which the quantity of active product released at the first usage is equal to 70% of the total quantity of active product contained in the material.

3. The material (10) according to Claim 1 or 2, in which the quantity of active product per unit of volume decreases in a linear fashion when there is movement away from the application surface (Fig. 2).

4. The material (10) according to Claim 1, 2 or 3, in which the quantity of microcapsules of the same size decreases in a linear fashion when there is movement away from the application surface (12).

5. The microcapsule support material according to one of Claims 1 to 4, in which said binder having a spongy consistency is a polymer foam containing at least 60% open cells.

6. The microcapsule support material according to Claim 5, in which said foam is a polyurethane foam.

7. A use of the microcapsule support material according to one of Claims 1 to 6, for obtaining a medicament for a cutaneous treatment using the product contained in said microcapsules.

8. A use of the microcapsule support material according to one of Claims 1 to 6, as an applicator device for a cosmetic product contained in said microcapsules.

9. A use of the microcapsule support material according to one of Claims 1 to 6, as a dispensing device for odoriferous effluvia and particularly perfume contained in said microcapsules.

10. A use of the microcapsule support material according to one of Claims 1 to 6, as a dispenser for soap contained in said microcapsules.

11. A capsule (22, 24, 26) containing a microcapsule support material for carrying out the use according to one of Claims 7 to 10.

12. The microcapsule support material (10) containing at least one active product not to be exposed to air, said microcapsules being disseminated in a solid binder having a spongy consistency such that the active product is released when pressure is exerted on the support material;
said material being **characterised in that** the quantity of microcapsules per unit of volume or the size of the microcapsules increases when there is movement away from the application surface (13), such that the quantity of active product per unit of volume increases as there is movement away from said application surface, thus enabling the quantity of active product released at said application surface to be almost constant at each usage.

13. The microcapsule support material according to one of Claims 1 to 6 or 12, in which some of the microcapsules contain a first product and the other microcapsules contain a second product, the two products reacting with one another when pressure is exerted on the support.

14. A use of the microcapsule support material according to Claim 13, as a glue resulting from a chemical reaction between the two products contained in said microcapsules.

## Patentansprüche

1. Mikrokapsel-Trägermaterial (10), das mindestens einen Wirkstoff, der unter Luftabschluss gehalten werden muss, enthält, wobei die Mikrokapseln in einem festen Bindemittel von schwammartiger Konsistenz so verstreut sind, dass der Wirkstoff freigesetzt wird, wenn Druck auf das Trägermaterial ausgeübt wird;
wobei das Material **dadurch gekennzeichnet ist, dass** sich beim Sich-Distanzieren von der Anwendungsfläche (12) entweder die Größe der Mikrokapseln reduziert oder die Menge der Mikrokapseln pro Raumeinheit so linear reduziert, dass sich die Menge des Wirkstoffs pro Raumeinheit beim sich nach und nach Distanzieren von der Anwendungsfläche reduziert, wodurch ermöglicht wird, dass die Menge des freigesetzten Wirkstoffs bei der Anwendungsfläche während der ersten Verwendung maximal ist.

2. Material (10) gemäß Anspruch 1, wobei die Menge des Wirkstoffs, der während der ersten Verwendung freigesetzt wird, 70 % der gesamten Menge des in dem Material enthaltenen Wirkstoffs beträgt.

3. Material (10) gemäß Anspruch 1 oder 2, wobei die Menge des Wirkstoffs pro Raumeinheit beim Sich-Distanzieren von der Anwendungsfläche (Fig. 2) linear abnimmt.

4. Material (10) gemäß Anspruch 1, 2 oder 3, wobei sich die Menge der Mikrokapseln von gleicher Größe beim Sich-Distanzieren von der Anwendungsfläche (12) linear reduziert.

5. Mikrokapsel-Trägermaterial gemäß einem der Ansprüche 1 bis 4,
wobei das Bindemittel von schwammartiger Konsistenz ein Polymerschaum, der mindestens 60 % offene Zellen enthält, ist.

6. Mikrokapsel-Trägermaterial gemäß Anspruch 5, wobei der Schaum ein Polyurethanschaum ist.

7. Die Verwendung des Mikrokapsel-Trägermaterials gemäß einem der Ansprüche 1 bis 6, um ein Medikament zu erhalten, das für eine Hautbehandlung, die das in den Mikrokapseln enthaltene Produkt verwendet, bestimmt ist.

8. Verwendung des Mikrokapsel-Trägermaterials gemäß einem der Ansprüche 1 bis 6 als Applikatorvorrichtung für ein kosmetisches Produkt, das in den Mikrokapseln enthalten ist.

9. Verwendung des Mikrokapsel-Trägermaterials gemäß einem der Ansprüche 1 bis 6 als Spendervorrichtung für wohlriechende Emanationen und insbesondere für Parfüm, das in den Mikrokapseln enthalten ist.

10. Verwendung des Mikrokapsel-Trägermaterials gemäß einem der Ansprüche 1 bis 6 als Spender von Seife, die in den Mikrokapseln enthalten ist.

11. Eine Kapsel (22, 24, 26), die ein Mikrokapsel-Trägermaterial enthält, um die Verwendung gemäß einem der Ansprüche 7 bis 10 einzusetzen.

12. Mikrokapsel-Trägermaterial (10), das mindestens einen Wirkstoff, der unter Luftabschluss gehalten werden muss, enthält, wobei die Mikrokapseln in einem festen Bindemittel von schwammartiger Konsistenz so verstreut sind, dass der Wirkstoff freigesetzt wird, wenn Druck auf das Trägermaterial ausgeübt wird;
wobei das Material **dadurch gekennzeichnet ist, dass** sich beim Sich-Distanzieren von der Anwendungsfläche (13) die Menge der Mikrokapseln pro Raumeinheit oder die Größe der Mikrokapseln so erhöht, dass sich die Menge des Wirkstoffs pro Raumeinheit beim sich nach und nach Distanzieren von der Anwendungsfläche vergrößert, wodurch ermöglicht wird, dass die Menge des freigesetzten Wirkstoffs bei der Anwendungsfläche für jede Verwendung ungefähr konstant ist.

13. Mikrokapsel-Trägermaterial gemäß einem der Ansprüche 1 bis 6 oder 12, wobei einige Mikrokapseln ein erstes Produkt enthalten und die anderen Mikrokapseln ein zweites Produkt enthalten, wobei die zwei Produkte eine Reaktion eingehen, wenn Druck auf den Träger ausgeübt wird.

14. Verwendung des Mikrokapsel-Trägermaterials gemäß Anspruch 13 als Klebstoff, der sich durch eine chemische Reaktion zwischen den beiden Produkten, die in den Mikrokapseln enthalten sind, ergibt.
